# EUROPEAN PATENT APPLICATION

(11) **EP 3 677 175 A1**
(43) Date of publication of application: **08.07.2020**
(21) Application number: 19150619.5
(22) Date of filing: 07.01.2019
(51) Int. Cl.: A61B 5/026, A61B 5/00

(54) **ARTERY SENSING**

(71) Applicant: Nokia Technologies Oy, 02610 Espoo (FI)
(72) Inventor: MÜLLER, Kiti, 00430 Helsinki (FI); LINDHOLM, Harri, 00320 Helsinki (FI); HIRVONEN, Antti, Sunnyvale, CA California 94086 (US); LAITIO, Mika, Sunnyvale, CA California 94086 (US)
(74) Representative: Whiting, Gary

(57) **Abstract**

An apparatus and method is described comprising: obtaining first measurement data relating to blood flow in a left vertebral artery of a subject from a first sensor of an apparatus applied to a neck of the subject; obtaining second measurement data relating to blood flow in a left carotid artery of the subject from a second sensor of the apparatus applied to the neck of the subject; obtaining third measurement data relating to blood flow in a right carotid artery of the subject from a third sensor applied to the neck of the subject; and obtaining fourth measurement data relating to blood flow in a right vertebral artery of the subject from a fourth sensor applied to the neck of the patient, wherein: the first to fourth measurement data are obtained in synchronism with each other; and each of the first to fourth measurement data includes one or more of pulse amplitude, blood flow velocity or blood flow quantity for the respective artery.

## Description

### Field

The present specification relates to the cervical artery sensing; in particular, to an apparatus, such as a collar, for measuring aspects of artery performance.

### Background

The brain receives blood from four main cervical arteries, consisting of two carotid arteries and two vertebral arteries. Although a number of solutions exist for measuring aspects of the performance of these arteries, there remains a need for alternative and improved arrangements.

### Summary

In a first aspect, this specification describes an apparatus comprising: first sensor means for obtaining first measurement data relating to blood flow in a left vertebral artery of a subject; second sensor means for obtaining second measurement data relating to blood flow in a left carotid artery of the subject; third sensor means for obtaining third measurement data relating to blood flow in a right carotid artery of the subject; and fourth sensor means for obtaining fourth measurement data relating to blood flow in a right vertebral artery of the subject, wherein: the first to fourth measurement data are obtained in synchronism with each other; and each of the first to fourth measurement data includes one or more of pulse amplitude, blood flow velocity or blood flow quantity for the respective artery. Each sensor means may, for example, be implement using piezoelectric or PPG sensors. Moreover, each sensor means may have a single sensor or multiple sensors.

At least some of said first to fourth sensor means may include an audio sensor and at least some of said first to fourth measurement data may include audio data relating to blood flow in the respective artery of the subject.

Some embodiments further comprise means (such as a video camera) for capturing image data.

Some embodiments further comprise means for determining an angle of head rotation and/or an angle of head tilt (e.g. relative to a base head rotation/ tilt angle) for the subject, which data may be obtained as "fifth measurement data" or may form part of the first to fourth measurement data described above. This maybe implemented using the means for capturing image data referred to above, such that the determining is based on captured image data. However, alternative implementation are possible, such as ultrasound or fusion imaging, in which the arterial flow dynamics data could be combined, for example, with neck brain area arteriography imaging and other imaging of cerebral flow.

There may be provided means for storing at least some of said measurement data. For example, in addition to (possibly synchronised) blood flow data from each sensor means, the measurement data may include some or all of: audio data relating to blood flow, head position information, head rotation angles, head tilt angles, image data etc.

Wireless transmission means may be provided for providing at least some of said measurement data to an external system.

The apparatus maybe a collar, such as a neck collar or a cervical collar. The collar may be formed from a stretchable and/or flexible material. This may be provided, for example, to enable a single collar to fit different sized subjects (with the same sensor placement positions).

In some embodiments, there may be provided means for positioning the apparatus relative to a neck of the subject. In this way, repeatable accurate placement of the apparatus may be enabled.

In some embodiments, a user interface may be provided for presenting at least some of said measurement data.

In a second aspect, this specification describes an apparatus comprising: means for obtaining first sensor data relating to blood flow in a left vertebral artery of a subject; means for obtaining second sensor data relating to blood flow in a left carotid artery of the subject; means for obtaining third sensor data relating to blood flow in a right carotid artery of the subject; means for obtaining fourth sensor data relating to blood flow in a right vertebral artery of the subject and means for generating measurement data based on said first to fourth sensor data and relative timing information relating to said data, wherein each of the first to fourth sensor data includes one or more of pulse amplitude, blood flow velocity or blood flow quantity for the respective artery. The apparatus may further comprise means for determining an angle of head rotation and/or an angle of head tilt for the subject from image data of said subject. A user interface may be provided for presenting at least some of said measurement data. There may be provided means for storing at least some of said sensor data.

In a third aspect, this specification describes a method comprising: obtaining first measurement data relating to blood flow in a left vertebral artery of a subject from a first sensor of an apparatus applied to a neck of the subject; obtaining second measurement data relating to blood flow in a left carotid artery of the subject from a second sensor of the apparatus applied to the neck of the subject; obtaining third measurement data relating to blood flow in a right carotid artery of the subject from a third sensor applied to the neck of the subject; and obtaining fourth measurement data relating to blood flow in a right vertebral artery of the subject from a fourth sensor applied to the neck of the patient, wherein: the first to fourth measurement data are obtained in synchronism with each other; and each of the first to fourth measurement data includes one or more of pulse amplitude, blood flow velocity or blood flow quantity for the respective artery. The method may further comprise storing time data together with said measurement data. The method may further comprising applying the apparatus to the neck of the subject such that said sensors are placed in position relative to said arteries and/or receiving a signal indicating that the apparatus has been applied to the subject.

At least some of said first to fourth sensors may include an audio sensor and at least some of said first to fourth measurement data may include audio data relating to blood flow in the respective artery of the subject.

Some embodiments further comprise receiving image data (for example from a video camera).

Some embodiments further comprise determining an angle of head rotation and/or an angle of head tilt (e.g. relative to a base head rotation/ tilt angle) for the subject, which data may be obtained as "fifth measurement data" or may form part of the first to fourth measurement data described above. This may be implemented based on captured image data.

The method may include storing at least some of said measurement data. Alternatively, or in addition, at least some of said measurement data may be transmitted (e.g. wirelessly) to an external system.

In a fourth aspect, this specification describes a method comprising: obtaining first sensor data relating to blood flow in a left vertebral artery of a subject; obtaining second sensor data relating to blood flow in a left carotid artery of the subject; obtaining third sensor data relating to blood flow in a right carotid artery of the subject; obtaining fourth sensor data relating to blood flow in a right vertebral artery of the subject and generating measurement data based on said first to fourth sensor data and relative timing information relating to said data, wherein each of the first to fourth sensor data includes one or more of pulse amplitude, blood flow velocity or blood flow quantity for the respective artery.

In a fifth aspect, this specification describes any apparatus configured to perform any method as described with reference to the third or fourth aspect.

In a sixth aspect, this specification describes computer-readable instructions which, when executed by computing apparatus, cause the computing apparatus to perform any method as described with reference to the third or fourth aspect.

In a seventh aspect, this specification describes a computer program comprising instructions for causing an apparatus to perform at least the following: obtain first measurement data relating to blood flow in a left vertebral artery of a subject from a first sensor of an apparatus applied to a neck of the subject; obtain second measurement data relating to blood flow in a left carotid artery of the subject from a second sensor of the apparatus applied to the neck of the subject; obtain third measurement data relating to blood flow in a right carotid artery of the subject from a third sensor applied to the neck of the subject; and obtain fourth measurement data relating to blood flow in a right vertebral artery of the subject from a fourth sensor applied to the neck of the patient, wherein: the first to fourth measurement data are obtained in synchronism with each other; and each of the first to fourth measurement data includes one or more of pulse amplitude, blood flow velocity or blood flow quantity for the respective artery.

In an eighth aspect, this specification describes a computer-readable medium (such as a non-transitory computer readable medium) comprising program instructions stored thereon for performing at least the following: obtaining first measurement data relating to blood flow in a left vertebral artery of a subject from a first sensor of an apparatus applied to a neck of the subject; obtaining second measurement data relating to blood flow in a left carotid artery of the subject from a second sensor of the apparatus applied to the neck of the subject; obtaining third measurement data relating to blood flow in a right carotid artery of the subject from a third sensor applied to the neck of the subject; and obtaining fourth measurement data relating to blood flow in a right vertebral artery of the subject from a fourth sensor applied to the neck of the patient, wherein: the first to fourth measurement data are obtained in synchronism with each other; and each of the first to fourth measurement data includes one or more of pulse amplitude, blood flow velocity or blood flow quantity for the respective artery. The method may further comprise storing time data together with said measurement data. The method may further comprising applying the apparatus to the neck of the subject such that said sensors are placed in position relative to said arteries and/or receiving a signal indicating that the apparatus has been applied to the subject.

In a ninth aspect, this specification describes an apparatus comprising: at least one processor; and at least one memory including computer program code which, when executed by the at least one processor, causes the apparatus to: obtain first measurement data relating to blood flow in a left vertebral artery of a subject from a first sensor of an apparatus applied to a neck of the subject; obtain second measurement data relating to blood flow in a left carotid artery of the subject from a second sensor of the apparatus applied to the neck of the subject; obtain third measurement data relating to blood flow in a right carotid artery of the subject from a third sensor applied to the neck of the subject; and obtain fourth measurement data relating to blood flow in a right vertebral artery of the subject from a fourth sensor applied to the neck of the patient, wherein: the first to fourth measurement data are obtained in synchronism with each other; and each of the first to fourth measurement data includes one or more of pulse amplitude, blood flow velocity or blood flow quantity for the respective artery.

In a tenth aspect, this specification describes an apparatus comprising: a first sensor for obtaining first measurement data relating to blood flow in a left vertebral artery of a subject; a second sensor for obtaining second measurement data relating to blood flow in a left carotid artery of the subject; a third sensor for obtaining third measurement data relating to blood flow in a right carotid artery of the subject; and a fourth sensor for obtaining fourth measurement data relating to blood flow in a right vertebral artery of the subject, wherein: the first to fourth measurement data are obtained in synchronism with each other; and each of the first to fourth measurement data includes one or more of pulse amplitude, blood flow velocity or blood flow quantity for the respective artery. Each sensor may, for example, be implement using piezoelectric or PPG sensors. Moreover, each sensor means may have a single sensor or multiple sensors.

### Brief description of the drawings

Example embodiments will now be described, by way of non-limiting examples, with reference to the following schematic drawings, in which:
FIGS. 1 and 2 are schematic diagrams of systems showing arteries feeding blood to a brain of a subject;
FIG. 3 is a collar in accordance with an example embodiment;
FIG. 4 is a block diagram of a collar system in accordance with an example embodiment;
FIG. 5 is a side view of a neck;
FIG. 6 is a side view of a neck showing sensor positions in accordance with an example embodiment;
FIG. 7 is a side view of a subject showing a collar positioned relative to the neck of the subject in accordance with an example embodiment;
FIG. 8 is a side view of a neck showing measurement locations in accordance with an example embodiment;
FIG. 9 is a flow chart showing an algorithm in accordance with an example embodiment;
FIG. 10 is a flow chart showing an algorithm in accordance with an example embodiment;
FIG. 11 shows head positions in accordance with an example embodiment;
FIG. 12 shows head positions in accordance with an example embodiment;
FIG. 13 is a block diagram of a system in accordance with an example embodiment;
FIG. 14 shows a user interface in accordance with an example embodiment;
FIG. 15 is a block diagram of a system in accordance with an example embodiment; and
FIGS. 16A and 16B show tangible media, respectively a removable memory unit and a compact disc (CD) storing computer-readable code which when run by a computer perform operations according to example embodiments.

### Detailed description

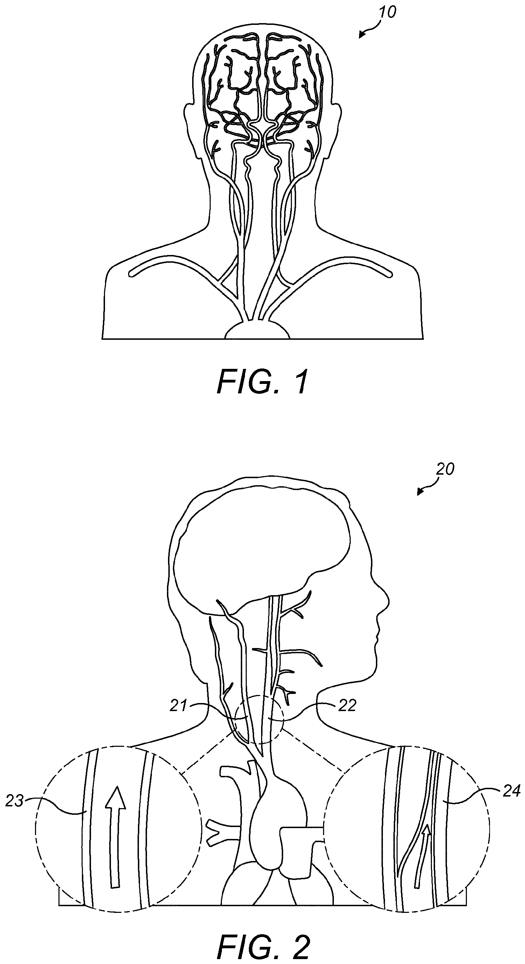

FIG. 1 is a schematic diagram of a system, indicated generally by the reference numeral 10, showing arteries feeding blood to a brain of a subject. As indicated schematically in the diagram 10, two major sets of vessels in the neck, named the cervical arteries, are the right and left carotid and vertebral arteries. These arteries supply blood to the brain.

The system 10 shows the basal area of the brain, where the vertebral arteries merge into the basilar artery. The basilar artery and the two carotid arteries feed blood into the Circle of Willis from which the main cerebral arteries originate. The Circle of Willis ensures adequate blood flow to the brain.

Dysfunction in one or several cervical arteries is one of the major underlying causes of brain stroke. Also, in cardiac disease attacks, blood flow to the brain can be disturbed. With ageing, arterial stiffness increases and the risk of transient ischemic arterial attacks (TIA) in the brain increases. Also, cardiac dysfunction may disturb blood flow in the cervical arteries.

TIA attacks can progress into an actual brain stroke. Thus, a TIA is a warning signal of increased risk of brain stroke. Thus solutions to evaluate the functions of the cervical arteries after a TIA attack are important for deciding needed treatment. Dizziness and vertigo are common symptoms and solutions to examine if the underlying cause is transient disturbed blood flow via cervical arteries into the brain due to e.g. neck motions are needed. Also, repeated TIA causes eventually vascular brain memory disease.

Early treatment of cervical artery disease can be hampered by lack of data relating to the functional state of such arteries. Accordingly, there is a need for systems that enable measurements of aspects of the performance of such arteries.

FIG. 2 is a schematic diagram of a system, indicated generally by the reference numeral 20, showing arteries feeding blood to a brain of the subject. Specifically, the system 20 shows a right vertebral artery 21 and a right carotid artery 22. (Left vertebral and carotid arteries are not visible in FIG. 2.)

The system 20 shows a first example artery 23 with normal blood flow and a second example artery 24 with restricted blood flow.

With age, elasticity of walls tends to decrease. The development of arterial stiffness can be accelerated by a wide range of illnesses (e.g. diabetes, elevated blood pressure, chronic inflammations etc.). In addition to stiffness, thickening of arterial walls (see the artery 24) reduces the diameter of the vascular pipe. In addition, local wall damage (e.g. plaques and erosion) can develop. These changes may cause turbulence in blood flow that can be detected by audio signals. Also, the amount of blood in the artery may decrease, which can be captured by measuring pulse amplitude and velocity in the arteries.

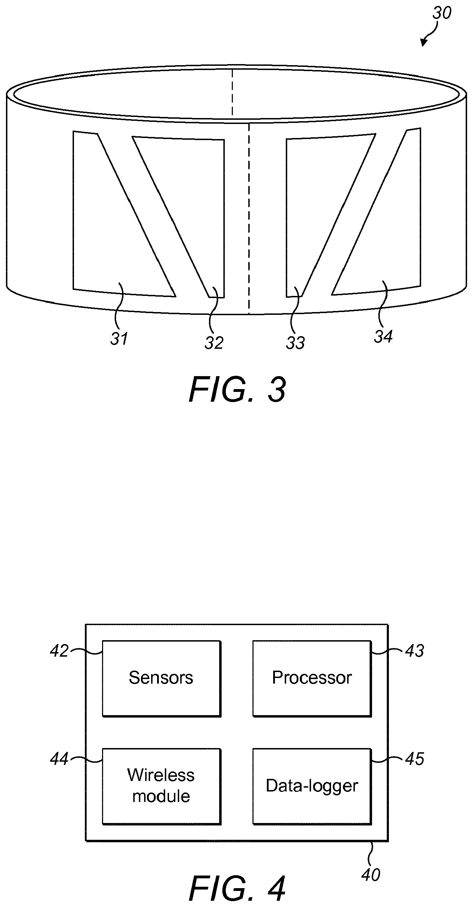

FIG. 3 is a collar, indicated generally by the reference numeral 30, in accordance with an example embodiment. The collar 30 includes a plurality of sensor means for obtaining measurement data relating to blood flow in the neck arteries. Specifically, the collar includes: first sensor means 31 for obtaining first measurement data relating to blood flow in a right vertebral artery of a subject; second sensor means 32 for obtaining second measurement data relating to blood flow in a right carotid artery of the subject; third sensor means 33 for obtaining third measurement data relating to blood flow in a left carotid artery of the subject; and fourth sensor means 34 for obtaining fourth measurement data relating to blood flow in a left vertebral artery of the subject. Each of said first to fourth sensor means 31 to 34 may comprise a single sensor, but could comprise multiple sensors. Each of the first to fourth measurement data includes one or more of pulse amplitude, blood flow velocity or blood flow quantity for the respective artery.

The sensor means may, for example, include piezoelectric and/or photoplethysmography (PPG) sensors (although other sensor configurations are possible in some example embodiments).

In some embodiments, the collar 30 is formed from a stretchable and/or flexible material. This may enable a single collar to fit differently sized subjects (with the same sensor placement positions relative to the posterior and carotid triangle described above).

As discussed in detail below, the collar 30 may be configured such that the first to fourth sensor means obtain measurement data in synchronism with each other. This may enable the measurement data to be compared and may enable an understanding of the status and dynamics of the blood flow to be developed. For example, by obtaining data for multiple arteries in synchronism with each other and/or relative to a time base, it may be possible to determine the presence of asymmetries and/or obstructions in the blood flow. Thus, for example, data from the first to fourth sensor means maybe generated with time stamp data, such that dynamic information regarding blood flows can be captured. If there is more than one sensor measuring flow in a particular artery (such as at an upper and lower part of the artery), information may be captured regarding flow (such as flow rates and changes in flow rates over time), for which time stamp data maybe desirable.

FIG. 4 is a block diagram of a collar system, indicated generally by the reference numeral 40, in accordance with an example embodiment. The collar system 40 comprises a plurality of sensors 42, a processor 43, a wireless module 44 and a memory module (e.g. data-logger) 45.

The sensors 42 may include the first to fourth sensor means 31 to 34 of the collar 30 described above. In addition, the sensors may include one or more audio sensors, such that the measurement data may include audio data relating to blood flow in the respective artery of the subject. Audio sensors may be provided, for example, to capture rushing sounds, sometimes referred to as bruits or vascular murmur, that can be a sign of arterial wall disease.

As discussed further below, the sensors 42 may include means for capturing image data (such as a video camera or some other imaging device). Thus, the measurement data may include such image data (such as head position information).

The wireless module 44 may be provided to enable at least some of said measurement data to be provided to an external system. The memory module 45 may be provided for storing at least some of said measurement data. Some embodiments may include a memory module or data-logger 45 that forms part of the collar 30, whereby data is initially stored locally at the collar and later sent to an external data platform (e.g. for analysis) by the wireless module 44.

The elements of the system 40 are not essential to all embodiments of the invention. For example, the wireless module 44 may be replaced with some other communication means, or may be omitted entirely (such that no mechanism for enabling measurement data to be provided to an external system is provided by the system 40). Alternatively, or in addition, the memory module 45 may be omitted.

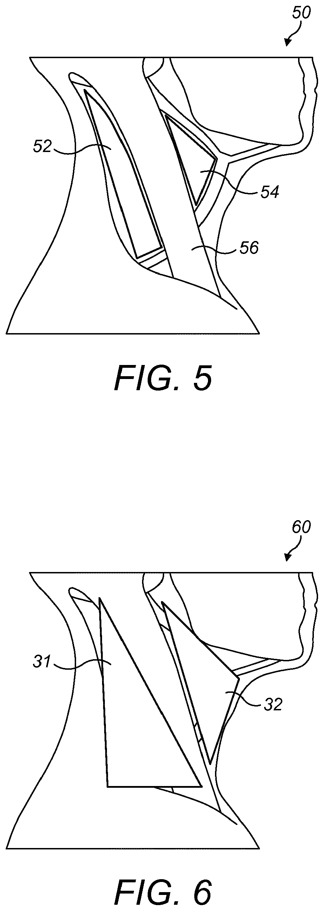

FIG. 5 is a side view of a neck, indicated generally by the reference numeral 50. The neck 50 shows a posterior lateral triangle sensing area 52 and a carotid triangle sensing area 54. The posterior and carotid triangle sensing areas 52 and 54 are separated by a sternocleidomastoid muscle 56, which can be detected with the human hand.

The sternocleidomastoid muscle and the ear mandibular corner can be used as upper rim landmarks for accurate placement of the sensors of the collar 30. The suboccipital triangle can be used as an additional landmark in the back of the head. Moreover, in some embodiments, the collar may be personalised to a particular user in such a way that on the upper and lower rim of the collar, markers are placed to ensure that the collar can be accurately positioned (e.g. where repeated measurements may need to be taken over an extended period of time). This may assist with ensuring that a patient, family member or medical professional can place the collar accurately. Furthermore, a place marker may be provided on the skin of the user to aid with accurate positioning.

FIG. 6 is a side view of a neck, indicated generally by the reference numeral 60. The neck 60 differs from the neck 50 by showing sensor positions in accordance with an example embodiment. More specifically, the first sensor means 31 (for obtaining first measurement data relating to blood flow in the right vertebral artery) is provided within the posterior lateral triangle sensing area 52 described above and the second sensor means 32 (for obtaining first measurement data relating to blood flow in the right carotid artery) is provided within the carotid-triangle sensing area 54 described above. It is possible that it is easier to identify carotid arteries than vertebral arteries. Thus, the positions of the carotid arteries may be of more relevance to positioning the collar (and the sensors) than the position of the vertebral arteries.

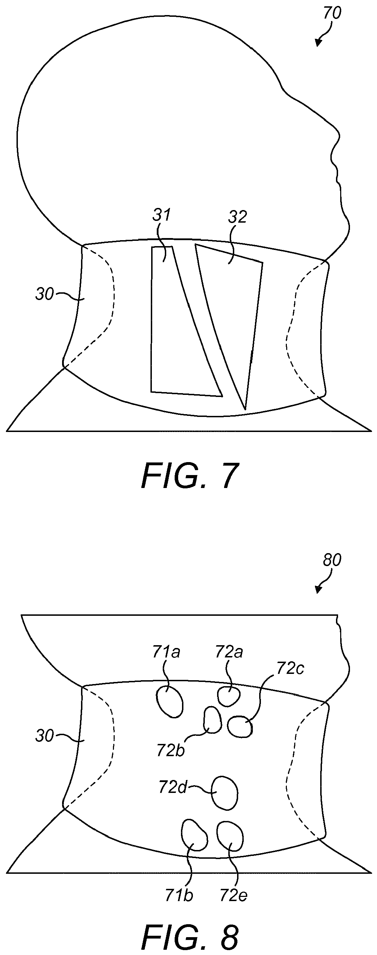

FIG. 7 is a side view of a subject, indicated generally by the reference numeral 70, showing a collar 30 positioned relative to the neck of the subject. The collar 30 is positioned such that the first sensor means 31 and the second sensor means 32 are aligned with the posterior and carotid triangle sensing areas 52 and 54 and the third and fourth sensor means 33 and 34 (not shown) are aligned with the relevant sensing areas on the left side of the head (also not shown).

FIG. 8 is a side view of a neck, indicated generally by the reference numeral 80, showing measurement locations in accordance with an example embodiment. A collar 30 is positioned such that the first sensor means 31 and the second sensor means 32 (not shown) are aligned with the posterior and carotid triangle sensing areas 52 and 54 (as described above).

A first measurement point 71a of the first sensor means and a second measurement 71b of the first sensor means are shown within the posterior triangle sensing area 52. The first and second measurement points 71a and 71b may be associated with different sensors that collectively form the first sensor means 31.

Similarly, a first measurement point 72a of the second sensor means, a second measurement 72b of the second sensor means, a third measurement point 72c of the second sensor means, a fourth measurement point 72d of the second sensor means, and a fifth measurement point 72e of the second sensor means are shown within the carotid triangle sensing area 54. The first to fifth measurement points 72a to 72e may be associated with different sensors that collected form the second sensor means 32.

Of course, the measurement points shown in FIG. 8 are provided by way of example only. Different numbers of measurement points could be provided as part of the various sensor means.

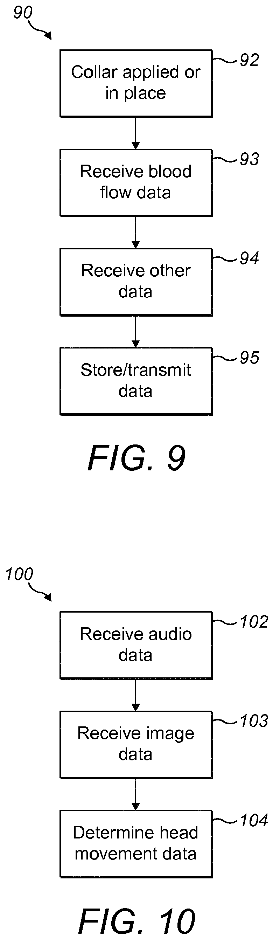

FIG. 9 is a flow chart showing an algorithm, indicated generally by the reference numeral 90, in accordance with an example embodiment.

The algorithm 90 starts at operation 92 where a collar 30 is applied to the neck of a subject (e.g. a patient) or a determination is made that the collar is in place (this may be relevant, for example, the collar was already in place). The operation 92 may include an arrangement for placing sensors (such as the sensor means 31 to 34) in position relative to the appropriate arteries (as discussed above). A determination that the collar is in place may, for example, involve receiving a signal that the collar is in place.

The operation 92 may be omitted is some embodiments.

At operation 93, blood flow data is received. For example, blood flow data may be received from some or all of the first to fourth sensors 31 to 34 described above. The blood flow data may include one or more of blood flow pulse amplitude, blood flow velocity and blood flow quantity data in the respective arteries. For example, in the configuration described above: first sensor data is obtained relating to blood flow in a left vertebral artery of a subject, second sensor data is obtained relating to blood flow in a left carotid artery of the subject; third sensor data is obtained relating to blood flow in a right carotid artery of the subject; and fourth sensor data is obtained relating to blood flow in a right vertebral artery of the subject.

At operation 94, other data may be received. As discussed further below, such other data may include audio and/or image data. In some embodiments, the operation 94 is omitted.

At operation 95, data is stored and/or transmitted. For example, data maybe transmitted using the wireless module 44 and/or stored at the memory module 45 of the system 40 described above.

At least some of the measurement data may include time data, such that time data may be stored and/or transmitted as part of the operation 95.

FIG. 10 is a flow chart showing an algorithm, indicated generally by the reference numeral 100, in accordance with an example embodiment. The algorithm 100 is an example implementation of the operation 94 discussed above with reference to FIG. 9.

At operation 102 of the algorithm 100, audio data may be received and, at operation 103, image data may be received. At operation 104, head movement data may be determined. As discussed further below, head movement data may be determined in the operation 104 based on image data obtained in the operation 103.

It should be noted that one or more of the operations 102 to 104 may be omitted and/or other data received in some example embodiments. Moreover, some of the operations of the algorithm 100 may be carried out in a different order.

Thus, the algorithms 90 and 100 may enable recording of arterial pulse amplitude, wave velocity and/or sounds produced by blood flow in the right and left vertebral artery and the right and left carotid artery of a subject, wherein the various data are recorded in synchronism with one another. A neck collar may be provided in which sensors can be located in area corresponding to the known anatomy of the neck area. Easily identifiable landmarks may be used to enable accurate placement of sensing areas of the collar.

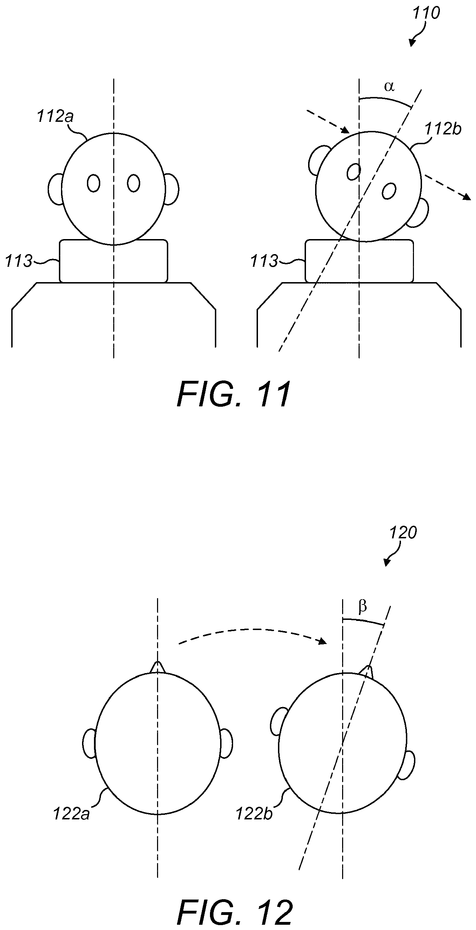

FIG. 11 shows head positions, indicated generally by the reference numeral 110, in accordance with an example embodiment. More specifically, a first head position 112a is shown in which a subject is in an upright position and a second head position 112b is shown in which the head of the subject is tilted to one side by an angle α. The position of a collar 113 of the subject is shown.

FIG. 12 shows head positions, indicated generally by the reference numeral 120, in accordance with an example embodiment. More specifically, a first head position 122a is shown in which the head of a subject is in a forward-facing direction and a second head position 122b is shown in which the head of the subject is rotated by an angle β.

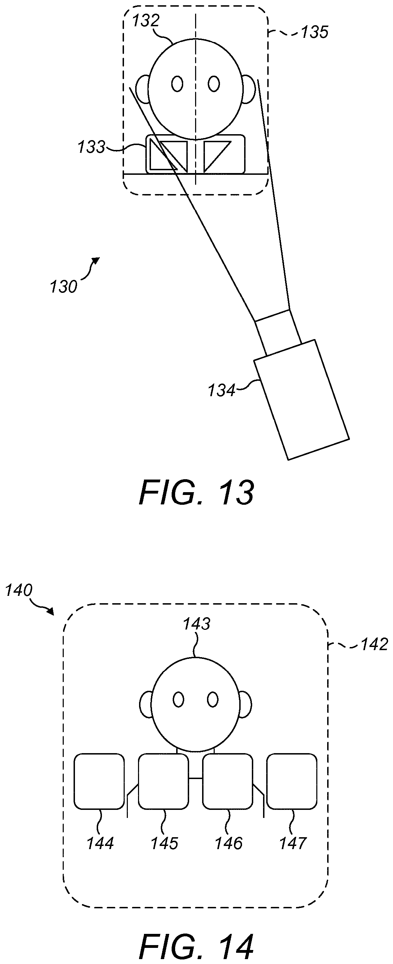

FIG. 13 is a block diagram of a system, indicated generally by the reference numeral 130, in accordance with an example embodiment. The system 130 comprises a subject 132 having a neck-mounted collar 133. The collar 133 includes the first to fourth sensors described above (shown schematically in the system 130).

The system 130 also comprises a device 134 (such as a video camera) for capturing image data. The image data may, for example, be used to determine an angle of head tilt (as shown in FIG. 11) and/or an angle of head rotation (as shown in FIG. 12). The angle of head tilt and/or the angle of head rotation may form part of the measurement data discussed above. The system 130 also shows an area 135 of an image formed by the imaging device 134.

Of course, other mechanisms could be provided for determining the angle of head tilt and/or the angle of head rotation, such as head mounted position sensors.

Example embodiments allow provocation examination to be carried out, for example in subjects with dizziness, to differentiate between neck muscle tension induced from arterial dysfunction related dizziness. For example, arterial function may be measured continuously with the head in different positions (e.g. side tilting, rotation, extension directly backward and obliquely to right and left). Moreover, such head position can be monitored in some example embodiments.

FIG. 14 shows a user interface, indicated generally by the reference numeral 140, in accordance with an example embodiment. The user interface includes an image area 142 that may match the area 135 described above with reference to the system 130.

The user interface 140 shows a subject 143 and four outputs 144 to 147. The four outputs 144 to 147 can be used to present plots of data obtained from the first to fourth sensors 31 to 34 described above. Thus, the outputs 144 to 147 can be used for presenting at least some of said measurement data described above.

The user interface 140 may form part of the collar 30, with basic information, such as timer information and sensor output traces being provided.

Of course, the user interface 140 is one of many possible user interfaces that could be provided in example embodiments.

Thus, example embodiments described herein may provide a number of technical effects, such as:
- The generation of time synchronised data on the status and key function of cervical (neck area) arteries (e.g. at the bedside). By way of example, the data may include strength of pulse, amount and velocity of blood flow in the vessel. The time synchronised data may enable investigation of how, for example, head position affects blood flow.
- Enabling longer-term recording of real-time measurements of arterial function during patient care (e.g. in hospital, at home and during remote consultations).
- Providing a tool with which to obtain information on how the functions of individual arteries affect the function of the other arteries responsible for providing blood to the brain.
- Allowing adequate, repeated placement of sensors at an optimal area of the neck of a subject for monitoring arterial function over an extended period of time (e.g. before and after medical procedures).

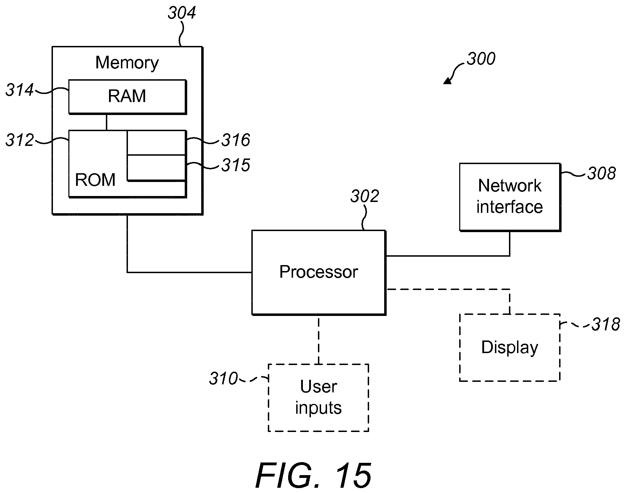

For completeness, FIG. 15 is a schematic diagram of components of one or more of the example embodiments described previously, which hereafter are referred to generically as processing systems 300. A processing system 300 may have a processor 302, a memory 304 closely coupled to the processor and comprised of a RAM 314 and ROM 312, and, optionally, user input 310 and a display 318. The processing system 300 may comprise one or more network/ apparatus interfaces 308 for connection to a network/ apparatus, e.g. a modem which maybe wired or wireless. Interface 308 may also operate as a connection to other apparatus such as device/ apparatus which is not network side apparatus. Thus, direct connection between devices/ apparatus without network participation is possible.

The processor 302 is connected to each of the other components in order to control operation thereof.

The memory 304 may comprise a non-volatile memory, such as a hard disk drive (HDD) or a solid-state drive (SSD). The ROM 312 of the memory 314 stores, amongst other things, an operating system 315 and may store software applications 316. The RAM 314 of the memory 304 is used by the processor 302 for the temporary storage of data. The operating system 315 may contain code which, when executed by the processor implements aspects of the algorithms 90 and 100 described above. Note that in the case of small device/ apparatus the memory can be most suitable for small size usage i.e. not always hard disk drive (HDD) or solid-state drive (SSD) is used.

The processor 302 may take any suitable form. For instance, it may be a microcontroller, a plurality of microcontrollers, a processor, or a plurality of processors.

The processing system 300 may be a standalone computer, a server, a console, or a network thereof. The processing system 300 and needed structural parts may be all inside device/ apparatus such as IoT device/ apparatus i.e. embedded to very small size

In some example embodiments, the processing system 300 may also be associated with external software applications. These may be applications stored on a remote server device/ apparatus and may run partly or exclusively on the remote server device/ apparatus. These applications maybe termed cloud-hosted applications. The processing system 300 may be in communication with the remote server device/ apparatus in order to utilize the software application stored there.

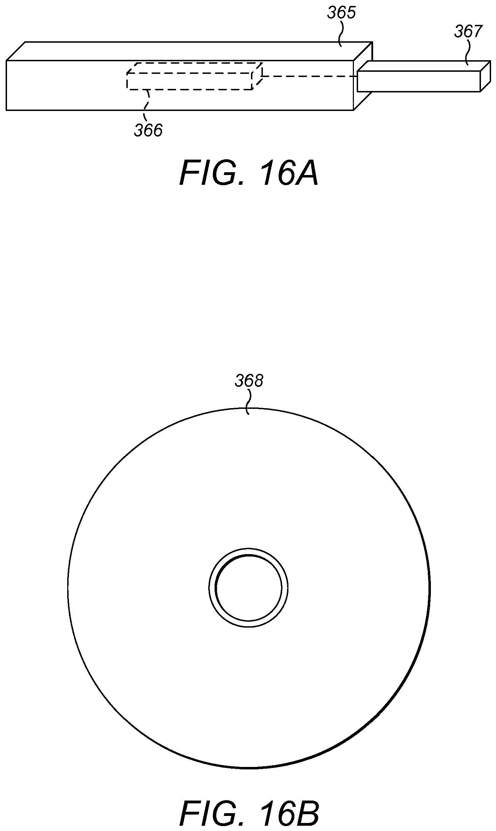

FIGS. 16A and 16B show tangible media, respectively a removable memory unit 365 and a compact disc (CD) 368, storing computer-readable code which when run by a computer may perform methods according to example embodiments described above. The removable memory unit 365 may be a memory stick, e.g. a USB memory stick, having internal memory 366 storing the computer-readable code. The memory 366 may be accessed by a computer system via a connector 367. The CD 368 may be a CD-ROM or a DVD or similar. Other forms of tangible storage media may be used. Tangible media can be any device/ apparatus capable of storing data/ information which data/ information can be exchanged between devices/ apparatus/ network.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" may be any non-transitory media or means that can contain, store, communicate, propagate or transport the instructions for use by or in connection with an instruction execution system, apparatus, or device, such as a computer.

Reference to, where relevant, "computer-readable storage medium", "computer program product", "tangibly embodied computer program" etc., or a "processor" or "processing circuitry" etc. should be understood to encompass not only computers having differing architectures such as single/multi-processor architectures and sequencers/parallel architectures, but also specialised circuits such as field programmable gate arrays FPGA, application specify circuits ASIC, signal processing devices/ apparatus and other devices/ apparatus. References to computer program, instructions, code etc. should be understood to express software for a programmable processor firmware such as the programmable content of a hardware device/ apparatus as instructions for a processor or configured or configuration settings for a fixed function device/ apparatus, gate array, programmable logic device/ apparatus, etc.

As used in this application, the term "circuitry" refers to all of the following: (a) hardware-only circuit implementations (such as implementations in only analogue and/or digital circuitry) and (b) to combinations of circuits and software (and/ or firmware), such as (as applicable): (i) to a combination of processor(s) or (ii) to portions of processor(s)/ software (including digital signal processor(s)), software, and memory(ies) that work together to cause an apparatus, such as a server, to perform various functions) and (c) to circuits, such as a microprocessor(s) or a portion of a microprocessor(s), that require software or firmware for operation, even if the software or firmware is not physically present.

If desired, the different functions discussed herein may be performed in a different order and/or concurrently with each other. Furthermore, if desired, one or more of the above-described functions may be optional or may be combined. Similarly, it will also be appreciated that the flow diagrams of Figures 9 and 10 are examples only and that various operations depicted therein maybe omitted, reordered and/or combined.

It will be appreciated that the above described example embodiments are purely illustrative and are not limiting on the scope of the invention. Other variations and modifications will be apparent to persons skilled in the art upon reading the present specification.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

## Claims

1. An apparatus comprising:
first sensor means for obtaining first measurement data relating to blood flow in a left vertebral artery of a subject;
second sensor means for obtaining second measurement data relating to blood flow in a left carotid artery of the subject;
third sensor means for obtaining third measurement data relating to blood flow in a right carotid artery of the subject; and
fourth sensor means for obtaining fourth measurement data relating to blood flow in a right vertebral artery of the subject,
wherein:
the first to fourth measurement data are obtained in synchronism with each other; and
each of the first to fourth measurement data includes one or more of pulse amplitude, blood flow velocity or blood flow quantity for the respective artery.

2. An apparatus as claimed in claim 1, wherein at least some of said first to fourth sensor means include an audio sensor and at least some of said first to fourth measurement data include audio data relating to blood flow in the respective artery of the subject.

3. An apparatus as claimed in claim 1 or claim 2, further comprising means for capturing image data.

4. An apparatus as claimed in any one of claims 1 to 3, further comprising means for determining an angle of head rotation and/or an angle of head tilt for the subject.

5. An apparatus as claimed in any one of the preceding claims, further comprising means for storing at least some of said measurement data.

6. An apparatus as claimed in any one of the preceding claims, further comprising wireless transmission means for providing at least some of said measurement data to an external system.

7. An apparatus as claimed in any one of the preceding claims, wherein said apparatus is a collar.

8. An apparatus as claimed in claim 7, wherein the collar is formed from a stretchable and/or flexible material.

9. An apparatus as claimed in any one of the preceding claims, further comprising means for positioning the apparatus relative to a neck of the subject.

10. An apparatus comprising:
means for obtaining first sensor data relating to blood flow in a left vertebral artery of a subject;
means for obtaining second sensor data relating to blood flow in a left carotid artery of the subject;
means for obtaining third sensor data relating to blood flow in a right carotid artery of the subject;
means for obtaining fourth sensor data relating to blood flow in a right vertebral artery of the subject and
means for generating measurement data based on said first to fourth sensor data and relative timing information relating to said data,
wherein each of the first to fourth sensor data includes one or more of pulse amplitude, blood flow velocity or blood flow quantity for the respective artery.

11. An apparatus as claimed in claim 10, further comprising means for determining an angle of head rotation and/or an angle of head tilt for the subject from image data of said subject.

12. An apparatus as claimed in any one of the preceding claims, further comprising a user interface for presenting at least some of said measurement data.

13. A method comprising:
obtaining first measurement data relating to blood flow in a left vertebral artery of a subject from a first sensor of an apparatus applied to a neck of the subject;
obtaining second measurement data relating to blood flow in a left carotid artery of the subject from a second sensor of the apparatus applied to the neck of the subject;
obtaining third measurement data relating to blood flow in a right carotid artery of the subject from a third sensor applied to the neck of the subject; and
obtaining fourth measurement data relating to blood flow in a right vertebral artery of the subject from a fourth sensor applied to the neck of the patient,
wherein:
the first to fourth measurement data are obtained in synchronism with each other; and
each of the first to fourth measurement data includes one or more of pulse amplitude, blood flow velocity or blood flow quantity for the respective artery.

14. A method as claimed in claim 13, further comprising storing time data together with said measurement data.

15. A method as claimed in claim 13 or claim 14, further comprising applying the apparatus to the neck of the subject such that said sensors are placed in position relative to said arteries and/or receiving a signal indicating that the apparatus has been applied to the subject.
